# EUROPÄISCHE PATENTSCHRIFT

(11) **EP 0 700 415 B1**
(45) Veröffentlichungstag und Bekanntmachung des Hinweises auf die Patenterteilung: **06.08.1997**
(21) Anmeldenummer: 93909540.2
(22) Anmeldetag: 03.05.1993
(51) Int. Cl.: C08L 1/28, C08L 3/00, C08L 5/00, A61L 15/28, A61L 15/60

(54) **POLYMERZUSAMMENSETZUNG, ABSORPTIONSMATERIALZUSAMMENSETZUNG, DEREN HERSTELLUNG UND VERWENDUNG**
POLYMER COMPOSITION, ABSORBENT MATERIAL COMPOSITION, THEIR PRODUCTION AND THEIR USE
COMPOSITION POLYMERE, COMPOSITION DE MATERIAU ABSORBANT, LEUR PRODUCTION ET LEUR UTILISATION

(43) Veröffentlichungstag der Anmeldung: 13.03.1996
(73) Patentinhaber: Stockhausen GmbH & Co. KG, 47805 Krefeld (DE)
(72) Erfinder: KLIMMEK, Helmut, D-4150 Krefeld (DE); GÜNTHER, Uwe, 75392 Deckenpfronn (DE); BRÜGGEMANN, Helmut, 47829 Krefeld (DE)
(74) Vertreter: Klöpsch, Gerald, Dr.-Ing. Patentanwalt
(86) Internationale Anmeldenummer: EP9301061
(87) Internationale Veröffentlichungsnummer: WO9425520

(56) Entgegenhaltungen:
- EP-A- 0 405 993
- EP-A- 0 481 225
- EP-A- 0 481 226
- DE-A- 4 206 856

## Beschreibung

Die Erfindung betrifft eine Polymerzusammensetzung und insbesondere Absorptionsmaterialien, die überwiegend auf nachwachsenden Rohstoffen basieren und somit grundsätzlich auch biologisch abbaubar sind. Aufgrund der überwiegend nativen Herkunft enthalten die Absorber keine oder deutlich geringere Mengen an Restmonomeren als Absorber auf Polyacrylatbasis. Die erfindungsgemäßen Polymerzusammensetzungen und Absorber besitzen eine vergleichsweise hohe Aufnahmekapazität und -geschwindigkeit für Wasser und wäßrige Lösungen, zeigen keine Neigung zum Gelblocking (Gelblocking: Beim Kontakt mit Wasser verkleben die äußeren Schichten des Absorbers und verhindern ein weiteres Vordringen der Flüssigkeit in den Absorber) und sind mechanisch stabil (bezüglich der Entmischung in die Einzelkomponenten). In gequollenem Zustand separieren sie in einzelne Partikel, sie sind nicht wäßrig und weisen eine sehr hohe Gelstabilität auf. Die Erfindung betrifft ferner ein Verfahren zu ihrer Herstellung und ihre Verwendung als Faser, Film, Pulver oder Granulat zur Absorption von Wasser, wäßrigen Lösungen oder wäßrigen Dispersionen und Körperflüssigkeiten in Hygienemitteln wie Tampons oder Windeln und Tierhygieneartikeln, in chemisch-technischen Produkten wie beispielsweise Verpackungsmaterialien insbesondere für Fleisch und Fisch, in Kulturgefäßen sowie zur Bodenverbesserung und als Kabelummantelungen.

Die meisten der heute verwendeten Absorptionsmaterialien, auch Superabsorber genannt, die in der Lage sind, in kurzer Zeit große Flüssigkeitenmengen (Wasser, Urin) aufzunehmen, stellen in erster Linie schwach vernetzte Polyacrylate dar und basieren somit nicht auf nachwachsenden Rohstoffen und sind vergleichsweise unzureichend bzw. überhaupt nicht biologisch abbaubar.

Im Bestreben, Superabsorber aus nachwachsenden Rohstoffen aufzubauen, wurde wie in der DE-C-2612846 beschrieben, Acrylsäure auf Polysaccharide, wie z.B. auf Maisstärke, aufgepfropft. Dabei können allerdings nur geringe Mengen an Polysacchariden (bis max. 25 %) eingesetzt werden, da ansonsten drastische Verschlechterungen der Absorptionseigenschaften erhalten werden.

Genauso können durch die Einarbeitung von Polysacchariden ins Polymerisationsgel von Polyacrylaten, wie in den DE-OS'en 40 29 591, 40 29 592 und 40 29 593 beschrieben wird, die Polyacrylate nur bis zu max. 25 % ersetzt werden, ohne eine deutliche Verschlechterung des Aufnahmevermögens und anderer Eigenschaften der resultierenden Superabsorber zu erhalten, auch wenn zusätzlich diverse Hilfsstoffe, wie Fasern und z.B. Aluminiumvernetzer zugesetzt werden. Die Polysaccharide werden als Bausteine für die Absorber gesehen, um biologisch abbaubare Einheiten zu erhalten.

Die DE-PS 3132976 beschreibt die Vermischung von Polyacrylsäure mit Polysacchariden in Pulverform und in Lösung, wobei die Hülle der Absorberteilchen der Gemische mit Aluminiumvernetzern, wie Al(OH)₂OOCCH₃ * 1/3 H₃BO₃ vernetzt wird. Demnach sind auch mit diesem Verfahren keine Superabsorber darstellbar, die zu über 60 % aus nachwachsenden Rohstoffen bestehen.

In den bislang nach dem heutigen Stand der Technik beschriebenen Verfahren besitzen die Polysaccharide keinen entscheidenen Beitrag als Absorptionskomponente.

Zahlreiche Patentveröffentlichungen wie die DE-OS 26 34 539 beschreiben die Herstellung von Carboxymethylcellulose-Absorbern, also von Materialien, die prinzipiell biologisch abbaubar sind, durch eine Vernetzung der Carboxymethylcellulose mit diversen Vernetzern im wäßrigen System. Diese Absorber zeigen allerdings ein starkes Gelblocking.

In der US-A 4 959 341 wird die Herstellung eines auf Carboxymethylcellulose basierenden Absorbers beschrieben,
der aus einer Mischung von Carboxymethylcellulose, Cellulosefasern, einer hydrophoben Komponente und Al(OH)₂OOCCH₃ * 1/3 H₃BO₃ als Vernetzer besteht, wobei der Aluminiumvernetzer eine Vernetzung der Carboxymethylcellulose während der Flüssigkeitsaufnahme bewirkt.
Diese Absorber besitzen gute Absorptionseigenschaften, zeigen aber Blockphänomene. Zudem werden diese Absorber durch mechanische Belastungen, wie Absiebung oder Förderung, leicht entmischt, so daß sie nicht mehr als homogenes Produkt vorliegen, was ihre Einsatzmöglichkeiten stark einschränkt.

In der EP-B 0 201 895 wird ebenfalls die Herstellung eines Absorbers, der auf Carboxymethylcellulose basiert, beschrieben. Allerdings wird bei der Herstellung dieser Absorber in einer wäßrigen Lösung gearbeitet, in der die Carboxymethylcellulose nur in geringer Konzentration vorliegt. Weiterhin werden bei der Herstellung größere Mengen an organischen Lösungsmitteln benötigt. Die Herstellung dieser Carboxymethycellulose-Absorber erfordert zudem einen hohen Zeitaufwand. Die Absorber selbst zeigen Blockphänomene und eine niedrige Gelstärke.

Der vorliegenden Erfindung lag die Aufgabe zugrunde, auf einfachem Wege eine Polymerzusammensetzung und eine Absorptionsmaterialzusammensetzung (im folgendem kurz Absorber genannt) bereitzustellen, der die oben beschriebenen Mängel nicht aufweist und der die folgende Eigenschaften besitzt:
a) Der Absorber soll überwiegend aus Komponenten nativen Ursprungs bestehen und damit grundsätzlich auch biologisch abbaubar sein.
b) Die Absorber sollen eine hohe mechanische Festigkeit aufweisen, sie dürfen sich beim Sieben oder z.B. bei einer Schneckenförderung nicht in ihre einzelnen Komponenten auftrennen.
c) Die Absorber sollen eine vergleichsweise hohe Aufnahmege schwindigkeit und -kapazität für Wasser und wäßrige Lösungen besitzen.
d) Der Gehalt an Restmonomeren soll deutlich geringer liegen als bei herkömmlichen Absorbern auf Basis von Polyacrylaten.
e) Die Absorber sollen im gequollenen Zustand eine sehr hohe Gelstabilität aufweisen; dabei sollen die Absorberkörner separiert, in einzelnen Partikeln vorliegen.
f) Sie dürfen nicht zum Gelblocking neigen.

Erfindungsgemäß erfolgt die Lösung dieser Aufgabe durch eine Polymerzusammensetzung und eine Absorptionsmittelzusammensetzung, die im wesentlichen aus vier Komponenten besteht:
- einer Komponente A, die auf speziellen nachwachsenden Rohstoffen basiert
- einer Komponente B, die aus einem speziellen wasserquellbaren Polymeren besteht
- einer Matrix und
- einem ionischen oder kovalenten Vernetzer sowie
- gegebenenfalls unter Zusatz eines Antiblockingmittels.

Die vorliegende Erfindung betrifft somit eine Polymerzusammensetzung, im wesentlichen bestehend aus 70 bis 99,9 Gew.-% wenigstens einer Komponente A aus wasserlöslichen Polysacchariden und deren Derivaten, die gegebenenfalls durch Vernetzung modifiziert worden sind (Komponente A)
und
0,1 bis 30 Gew.-% wenigstens einer Komponente B aus wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und /oder Copolymere durch eine mindestens bifunktionelle Verbindung vernetzt sind (Komponente B)
sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder covalenten Vernetzers
und
O bis 50 Gew.-%, bezogen auf die polymeren Komponenten A plus B, wenigstens eines Antiblockingmittels auf Basis natürlicher und/oder synthetischer Fasern und/oder Materialien mit großer Oberfläche.

Weiter betrifft die vorliegende Erfindung eine wirkstoffenthaltende Zusammensetzung der obigen Art, die wenigstens einen Wirkstoff, z. B. ein Arzneimittel, ein Pestizit, ein Bakterizid und/oder einen Riechstoff enthält, wobei der Wirkstoff verzögert freigesetzt wird.

Dabei wurde überraschenderweise gefunden, daß ein geringfügiger Zusatz von Komponente B zur Komponente A eine deutliche Verbesserung der Absorptionseigenschaften bewirkt. Da nur geringe Zusätze an Komponente B notwendig sind, liegt damit der Restmonomerengehalt an z. B. Acrylsäure eines solchen Absorbers deutlich geringer als bei Absorbern auf Polyacrylatbasis.
Ferner wurde überraschenderweise gefunden, daß durch den Zusatz eines Feststoffes, der als Matrix für das Absorbersystem fungiert, in Kombination mit dem Polymerabsorptionsmittel, einer Mischung aus den Komponenten A und B, und einem ionischen Vernetzer und ggf. eines Abtiblockingmittels, ein Absorptionsmittel herstellbar ist, das eine hohe Aufnahmegeschwindigkeit und -kapazität für Wasser und wässrige Lösungen sowie eine verbesserte mechanische Festigkeit hinsichtlich der Entmischung der einzelnen trockenen Partikel besitzt. Weiterhin liegen die Gele dieses Absorbersystems separiert in einzelnen Partikeln vor.

Außerdem zeigen diese Absorber überraschenderweise, in Kombination mit den oben genannten Eigenschaften, eine Gelstärke, die deutlich höher liegt, als die von Absorbern, die auf Polyacrylsäurebasis aufbauen.

Als Komponente A sind wasserlösliche und wasserquellbare Polymere auf der Basis von Polysacchariden und deren Derivate geeignet, wie Guar, Carboxymethylguar, Xanthan, Alginate, Gummi Arabicum, Hydroxyethyl- oder Hydroxypropylcellulose, Carboxymethylcellulose und andere Cellulosederivate, Stärke und Stärkederivate wie Carboxymethylstärke und Mischungen aus den einzelnen Polysacchariden. Die bevorzugten Polymeren sind Stärke, Guar und Cellulose sowie die anionischen Derivate von Stärke, Guar und Cellulose, wobei Carboxymethylcellulose ein besonders bevorzugtes Material darstellt.

Die aufgeführten Polymeren der Komponente A können durch eine Vernetzung modifiziert werden, um ihre Wasserlöslichkeit zu reduzieren und bessere Quelleigenschaften zu erreichen. Die Vernetzung kann sowohl im gesamten Polymeren stattfinden oder aber auch nur an der Oberfläche der einzelnen Polymerpartikel.

Die Umsetzung der Polymeren kann mit ionischen Vernetzern wie z.B. Calcium-, Aluminium-, Zirkon-, Eisen (III)- und Titanverbindungen erfolgen. Ebenso ist die Umsetzung mit polyfunktionellen Carbonsäuren wie Citronensäure, Schleimsäure, Weinsäure, Äpfelsäure, Malonsäure, Bernsteinsäure, Glutarsäure, Adipinsäure, mit Alkoholen wie Polyethylenglykole, Glycerin, Pentaerythrit, Propandiole, Saccharose, mit Kohlensäureestern wie Ethylen- und Propylencarbonat, mit Aminen wie Polyoxypropylenamine, mit Epoxiverbindungen wie Ethylenglykoldiglyci dylether, Glykoldi- oder triglycidylether und Epichlorhydrin, mit Säureanhydriden wie Bernsteinsäureanhydrid und Maleinsäureanhydrid, mit Aldehyden und mehrfunktionellen (aktivierten) Olefinen wie Bis-(acrylamido)-essigsäure und Methylenbisacrylamid möglich. Ebenso kommen Derivate der genannten Verbindungsklassen in Betracht sowie heterofunktionelle Verbindungen mit verschiedenen funktionellen Gruppen der oben genannten Verbindungsklassen.

Als Komponente B sind wasserquellbare synthetische Polymere oder Copolymere in erster Linie auf der Basis von (Meth-) Acrylsäure und weiterhin auf der Basis von (Meth-) Acrylnitril, (Meth-) Acrylamid, Vinylacetat, Vinylpyrrolidon, Vinylpyridin, Maleinsäure, Maleinsäureanhydrid, Itakonsäure, Itakonsäureanhydrid, Fumarsäure, Vinylsulfonsäure, 2-Acrylamido-2-methylpropansulfonsäure, sowie die Amide, ihre N-Alkyl- und N,N'-Dialkylderivate, hydroxylgruppenhaltige Ester und aminogruppenhaltige Ester der polymerisationsfähigen Säuren geeignet. Bevorzugt sind vernetzte, teilweise neutralisierte Polyacrylate.

Es können bis zu 98 %, vorzugsweise 50 - 80 % der Säuregruppen neutralisiert sein.

Die Polymeren können durch einen mindestens bifunktionellen Vernetzer vernetzt sein.

Die Herstellung dieser vorstehenden Polymeren erfolgt nach bekannten Verfahren (DE-C 27 06 135, DE-A 40 15 085). Ein besonders bevorzugtes Material als Komponente B stellen Polyacrylate dar, z.B. die von der Chemischen Fabrik Stockhausen GmbH hergestellten FAVOR^{R} Typen, die Gegenstand der DE-A 40 15 085 sind.

Als Matrix sind organische Feststoffe geeignet, die unter 180°C schmelzen bzw. erweichen und vorzugsweise bei Raumtemperatur eine weiche Konsistenz besitzen, wie z. B. Triglycerinmonostearat oder spezielle Wachsester. Ebenso sind hochviskose Flüssigkeiten wie z. B. Rizinusöl geeignet.
Vorzugsweise sind als Matrix Polycaprolactone geeigent, wie TONE 0230 und 0240 von Union Carbide, die auch modifiziert sein können, wie z.B. durch eine Umsetzung mit Maleinsäureanhydrid.

Durch die Matrix erhält das Absorbersystem vermutlich durch chemische und/oder physikalische Wechselwirkungen eine höhere mechanische Festigkeit, wodurch die Entmischung der Einzelkomponenten durch Transportvorgänge, wie z.B. mittels einer Förderschnecke, oder durch Absiebvorgänge stark vermindert wird. Dadurch kann ein Absorptionsmittel hergestellt werden, das nicht nur hohe Absorptionswerte besitzt, sondern nach einer Konfektionierung bzw. nach Einarbeitung in seinem Verwendungsbereich als homogeneres und somit wirksameres System vorliegt.

Außerdem bewirkt das Einbetten des Absorptionsmaterials in der Matrix überraschenderweise eine deutliche Reduzierung bzw. eine gänzliche Beseitigung des Gelblocking, somit ist eine hohe Aufnahmegeschwindigkeit im ganzen Absorber gewährleistet. Weiterhin wird durch die Matrix der Vernetzer fest an der Oberfläche der einzelnen Absorberpartikel fixiert. Die Granulierung von Superabsorberfeinstäuben durch Agglomerierungshilfsmittel wird in den Beispielen der DE-PS 37 41 157 und DE-PS 39 17 646 beschrieben. Die so hergestellten Produkte besitzen eine hohe Aufnahmegeschwindigkeit für Wasser und wäßrige Lösungen. Diese Produkte bestehen jedoch nur aus Polyacrylaten und sind folglich vergleichsweise unzureichend bzw. überhaupt nicht biologisch abbaubar. Die Agglomeriesationsmittel dienen nur zur Granulierung eines Produktes und keinesfalls als Matrixmaterial.

Die Anti-Blocking-Mittel vermindern ebenfalls Gelblocking; sie bewirken also eine schnellere und bessere Flüssigkeits aufnahme und sorgen dafür, daß die Gele separiert, d.h. in einzelnen Partikeln vorliegen.
Geeignete Anti-Blocking-Mittel sind bekanntermaßen (vgl. DE-PS 31 41 098 und DE-PS-33 13 344) Fasermaterialien und andere Materialien mit großer Oberfläche.
Die Fasern können natürliche oder synthetische Fasern sein, wie z.B. Woll-, Baumwoll-, Seiden- und Cellulosefasern, bzw. Polyamid-, Polyester-, Polyacrylnitril-, Polyurethanfasern, Fasern von Olefinen und deren Substitutionsprodukten sowie Polyvinylalkoholfasern und deren Derivate. Beipiele für anorganische Materialien sind Bentonite, Zeolithe, Aerosile und Aktivkohlen.

Geeignete Vernetzer sind Verbindungen, welche die oben beschriebenen Polymeren in einen Zustand überführen, in dem die Wasserlöslichkeit reduziert wird, das Saugvermögen verbessert und die Blockphänomene vermindert werden.

Als ionische Vernetzer sind Metallverbindungen geeignet, die mit den funktionellen Gruppen der Polymere in Wechselwirkung treten können. Besonders bevorzugt sind Magnesium-, Calcium, Aluminium-, Zirkon-, Eisen-, Titan- und Zinkverbindungen, die eine sehr gute Wasserlöslichkeit besitzen, wie die Salze von Carbonsäuren und anorganischen Säuren.
Bevorzugte Carbonsäuren sind Essigsäure, Milchsäure, Salicylsäure, Propionsäure, Benzoesäure, Fettsäuren, Malonsäure, Bernsteinsäure, Glutarsäure, Adipinsäure, Citronensäure, Weinsäure, Äpfelsäure und Schleimsäure.
Bevorzugte anorganische Anionen sind Chloride, Bromide, Hydrogensulfate, Sulfate, Phosphate, Borate, Nitrate, Hydrogencarbonate und Carbonate.
Weiterhin sind organische Verbindungen geeignet, die mehrwertige Metalle enthalten, wie Acetylacetonate und Alkoholate, wie z.B. Fe(acac)₃, Zr(acac)₄, Ti(OBu)₄ und Zr(o-Prop)₄.

Der wasserlösliche Vernetzer bewirkt eine Vernetzung der Komponenten A und B, sowohl untereinander als auch zwischeneinander, besonders an der Oberfläche, wodurch, wie in den DE-OS 31 32 976, DE-C 26 09 144 und der US-A 4 959 341 beschrieben ist, die Absorptionseigenschaften verbessert werden.

Als kovalente Vernetzer sind polyfunktionelle Carbonsäuren, Alkohole, Amine, Epoxiverbindungen, Carbonsäureanhydride und Aldehyde ,sowie deren Derivate, geeignet. Beispiele sind Citronensäure, Schleimsäure, Weinsäure, Äpfelsäure, Malonsäure, Bernsteinsäure, Glutarsäure, Adipinsäure, Polyethylenglykole, Glycerin, Propandiole, Polyoxypropylenamine, Epichlorhydrin, Ethylenglykoldiglycidylether, Glykoldiglycidyl ether, Bernsteinsäureanhydrid, Maleinsäureanhydrid, Ethylencarbonat und Propylencarbonat geeignet. Ebenso kommen natürlich Derivate der genannten Verbindungen in Betracht sowie heterofunktionelle Verbindungen mit verschiedenen funktionellen Gruppen der oben genannten Verbindungsklassen.

Der Anteil an Komponente A an dem Verhältnis Komponente A zu Komponente B beträgt 70 - 99,9 Gew.%, vorzugsweise 75 - 90 Gew.%. Der Anteil an Komponente B beträgt 0,1 - 30 Gew,% vorzugsweise 10 - 25 Gew.%.

Der Zusatz, auch in geringen Mengen, an Komponente B bewirkt eine starke Verbesserung der Absorptionseigenschaften, vor allem des Saugvermögens. Dadurch kann eine überraschend deutliche Verbesserung der Absorptionseigenschaften gegenüber reinem Carboxymethylcellulose-Material (CMC-Material) erzielt werden.

Die Menge an Anti-Blocking-Mittel beträgt vorzugsweise zwischen 0,5 und 50 Gew. %, besonders bevorzugt 5 - 15 Gew. %, bezogen auf die Komponenten A und B.

Die Vernetzermenge im Absorber beträgt 0,001 - 10 Gew.%, vorzugsweise 3 - 7 Gew.%, bezogen auf die Komponenten A und B.

Der Zusatz von Matrixmaterial bezogen auf die Komponenten A und B soll zwischen 0,1 - 30 Gew.%, vorzugsweise zwischen 2,5 und 7,5 Gew.% betragen.
Die Matrix verhindert ein Auseinanderfallen des Absorptionsmaterials, wie es bei reinen physikalischen Mischungen wie z.B. in der US-A 4 952 550 beobachtet wird und verhindert zusätzlich ein Gelblocking.

Die vorzugsweise Herstellung des Absorptionsmaterials wird nachfolgend beschrieben.
Zur Herstellung des erfindungsgemäßen Absorptionsmittels werden die Komponente A und die Komponente B physikalisch in trockener Form bei Raumtemperatur vermischt. Dieses Material wird mit dem Anti-Blocking-Mittel und der Matrix-Komponente vermischt, bis eine homogene Mischung vorliegt. Die Mischung der Komponenten erfolgt in geeigneten Mischern wie Schneckenmischer, Wirbelschichtbettmischer, Scheibenmischer oder Bandmischer.

Die Wärmebehandlung findet bei 25 - 180°C, vorzugsweise bei 100 - 120°C statt. Die Erwärmungsdauer beträgt 5 - 60 Minuten, vorzugsweise 20 - 40 Minuten. Zur Wärmebehandlung des Produktes werden gewöhnliche Trockner oder Heizöfen (z.B. Scheibentrockner, Bandtrockner, Wirbelschichtbetttrockner oder Infrarottrockner) eingesetzt.
Anschließend wird der ionische Vernetzer, vorzugsweise Aluminiumdihydroxyacetat, stabilisiert mit Borsäure, bei Raumtemperatur eingearbeitet, bis eine homogene Mischung entstanden ist. Zur Fixierung des Vernetzers durch die Matrix wird nochmals auf 25 - 180°C, vorzugsweise auf 50 - 80°C, für 5 - 60 Minuten erhitzt, um das Matrixmaterial aufzuschmelzen.

Die Komponenten A und B können vor der Vermischung abgesiebt werden, vorzugsweise im Bereich von 90 - 630 µm.
Die Einarbeitung der Matrixkomponenten erfolgt vorzugsweise bei Raumtemperatur, die Matrixkomponente kann aber auch als Schmelze eingesetzt werden.
Die Mischung kann vor der Wärmebehandlung mit einem vorzugsweise Wasser/Isopropanol-Gemisch versetzt werden, um einen Lösungsvermittler zu haben, der eine thermische Modifizierung der Komponente A, also einem Polysaccharid und nicht von Polyacrylsäure, untereinander, wie auch mit der Matrixkomponente und der Komponente B in den Randbereichen der Komponente A bewirkt, was sich positiv auf das Saugvermögen des Absorptionsmaterials auswirkt. Statt dem Wasser/Isopropanol-Gemisch können auch Wasser und andere Gemische von Wasser mit wasserlöslichen organischen Lösungsmitteln eingesetzt werden.

In der EP-PS 0 083 022 wird die Vernetzung eines Absorbers beschrieben, der aus Polyacrylsäure besteht, mit Vernetzern, die wenigstens zwei funktionelle Gruppen enthalten und in der Lage sind, mit den Carboxylgruppen des Polyacrylats zu reagieren. Die Reaktion erfolgt an der Oberfläche der Absorberteilchen.

DE-PS 33 14 019 und DE-PS 35 23 617 beschreiben ebenfalls die Oberflächenvernetzung von Polyacrylaten mit Hilfe von Vernetzern, die wenigstens zwei funktionelle Gruppen besitzen. Im Gegensatz zu den erfindungsgemäßen Absorbern werden in diesen Patenten nur Modifizierungen von Polyacrylaten, jedoch nicht von Polysacchariden, in der Hülle beschrieben, wodurch aber keinsfalls Absorber erhalten werden, die ausreichend biologisch abbaubar sind.
Die Einarbeitung des ionischen Vernetzers kann auch direkt in die physikalische Mischung Komponente A, Komponente B, Anti-Blocking-Mittel und Matrix-Material erfolgen, worauf dann auf 25 - 180°C, vorzugsweise auf 100 - 120°C, für 5 - 120 Minuten, vorzugsweise für 20 - 60 Minuten erwärmt wird.

Der oben beschriebene Lösungsmittel-Schritt kann bei diesem Verfahren vor oder nach der Vernetzereinarbeitung erfolgen.

Der kovalente Vernetzer kann alternativ und zusätzlich zu dem ionischen Vernetzer zu der Polymermischung vor oder nach der Matrixzugabe zugesetzt werden.
Der kovalente Vernetzer wird in einem vorzugsweise gegebenfalls Alkohol/Wasser-Gemisch gelost und der Polymermischung unter schnellem Rühren zugetropft. Die Lösungsmittel-Menge beträgt zwischen 1 und 10 % bezogen auf die Polymermischung. Anschließend wird auf 25 - 180°C für 5 - 120 Minuten erwärmt. Als Lösungsmittel können Wasser und Gemische aus Wasser mit wasserlöslichen organischen Lösungsmitteln verwendet werden.

Das erfindungsgemäße Absorptionsmaterial zeigt eine gute biologische Abbaubarkeit gegenüber Produkten, die auf einer Polyacrylsäurebasis beruhen, bei einem, gegenüber bisher bekannten Absorptionsmaterialien auf nativer Basis stark verbesserten Aufnahme- und Saugvermögen für eine 0,9 %ige Kochsalzlösung, bei einer überraschend sehr hohen Gelstärke.

| Gelstärke einiger erfindungsgemäßer Absorber sowie einiger marktbekannter Absorber | |
|---|---|
| Produktbezeichnung | Gelstärke (10 Hz) (N/m²) |
| erfindungsgemäße Absorber | |
| Superabsorber aus Bsp. 3 | ≥ 10000 |
| Superabsorber aus Bsp. 4 | ≥ 10000 |
| Superabsorber aus Bsp. 10 | ≥ 10000 |
| Superabsorber aus Bsp. 11 | ≥ 10000 |
| Superabsorber aus Bsp. 14 | ≥ 10000 |
| Superabsorber aus Bsp. 15 | ≥ 10000 |

| marktbekannte Absorber | |
|---|---|
| Produkt A | 2450 |
| Produkt B | 4200 |
| Produkt C | 3500 |
| Produkt D | 2700 |
| Produkt E | 4950 |
| Produkt F | 3700 |
| Produkt G | 1575 |
| Produkte A, B, C, D, F und G: vernetzte, teilweise neutralisierte Polyacrylate Produkt E: vernetztes, teilweise neutralisiertes Polyacrylat-Stärke-Pfropfpolymer. | |

Weiterhin ist die mechanische Festigkeit (in Bezug auf Entmischung in die Einzelkomponenten) gegenüber den zuvor beschriebenen, auf nachwachsenden Rohstoffen basierenden Absorbern deutlich verbessert.

Die erfindungsgemäße Polymerzusammensetzung kann insbesondere als Absorptionsmaterial als Faser, Film, Pulver oder Granulat eingesetzt werden, um Wasser oder wässrige Flüssigkeiten, wie Urin und Blut, aufzunehmen und ist somit besonders für den Einsatz in Windeln, Tampons, chirurgischen Erzeugnissen, Kabelummantelungen, Kulturgefäßen, Verpackungsmaterialien für Fleisch oder Fisch und absorbierenden Kleidungsstücken geeignet.

Außerdem ist das Material als Speichermedium zur sukzessiven Freisetzung von Wirkstoffen, wie Arzneimitteln, Pestiziden (US 4,818,534; US 4,983,389; US 4,983,390; US 4,985,251) und Riechstoffen geeignet, mit dem Vorteil, daß das Speichermedium abbaubar ist. Dadurch ergibt sich als weiterer Vorteil, daß der Wirkstoff vollständig freigesetzt wird.

Die wirkstoffhaltigen Depotmaterialien können durch Absorption, vorzugsweise konzentrierter, wässriger oder wasserhaltiger Lösungen in den weitgehend trockenen Absorber und ggf. dessen erneute Trocknung hergestellt werden.

Der Wirkstoff kann auch direkt oder als Lösung oder Dispersion bei beliebigen Vorstufen des Herstellungsprozesses der Absorberzusammensetzung zugesetzt werden.

Die wirkstoffhaltigen Depotmaterialien werden in Pulverform oder als Dispersion in hydrophoben Medien, die dispersionsstabilisierende Mittel wie Emulgatoren oder Stabilisatoren enthalten können, oder in Mischung mit anderen Stoffen wie Polysacchariden verwendet.

Beispielsweise wird durch den Zusatz solcher bakterizidhaltiger Depotmaterialien zu Cellulose-, Guar- oder Stärkeprodukten oder deren Derivate, wie Carboxymethylcellulose, bei deren Lagerung und Anwendung in wässrigen Medien der Abbau dieser Substanzen über längere Zeit verhindert und wobei durch die Depotwirkung größere Mengen von freiem Wirkstoff in der Lösung vermieden werden.

### Testmethoden:

### Teebeutel-Test (TBT)

Zur Bestimmung des Absorptionsvermögens wurde ein Teebeutel-Test durchgeführt. Als Prüflösung wurde eine wäßrige 0,9 %ige NaCl-Lösung verwendet.

0,2 g einer Prüfsubstanz (abgesiebt zwischen 90 und 630 µm), die in einem Teebeutel eingewogen wurde, ließ man 10 bzw. 30 Minuten in der Prüflösung aufquellen. Nach fünfminütigem Abtropfen (Maximalwert) wurde in einer Zentrifuge, wie z.B. in einer handelsüblichen Wäscheschleuder, bei 1400 Upm abgeschleudert. Die Flüssigkeitsaufnahme wurde gravimetrisch ermittelt und auf 1 g Substanz umgerechnet. (Retentionswert).

### Absorption under Load (AUL)

Um das Flüssigkeitsaufnahmevermögen unter Druck zu bestimmen, wurde die Absorption under Load , wie in EP-A-0 339 461 beschrieben, bestimmt.
0,16 g Prüfsubstanz (abgesiebt zwischen 300 und 600 µm) ließ man durch Kapillarwirkung für 60 Minuten unter einem Druck von 1,55 k N/m»( 99,8 g/in²) in 0,9 %ige NaCl-Lösung quellen. Die Flüssigkeitsaufnahme wurde gravimetrisch ermittelt und auf 1 g Substanz umgerechnet.

### Gelstärke (G')

Um die Gelstärke G' der gequollenen Absorber zu bestimmen, wurde, wie in EP-A 0 339 461 beschrieben, vorgegangen. Gerät: Controlled Stress Rheometer CS 100, Carri-Med Ltd. Dorking/UK.
Meßbedingungen: Plate-Platte-System, Durchmesser 60mm, Plattenabstand 2 mm, Temperatur 20°C, Drehmoment 1000 - 4000 µNm, Amplitude 1,5 - 5 mrad, Frequenz 10,0 Hz, 28 ml 0,9 % NaCl/g Absorber. Die Angaben erfolgen in N/m².

### Fließtest (FT)

Mittels des Fließtests wurde ermittelt, wie schnell die Produkte die Testflüssigkeit aufnahmen, ob sie Blockphänomene zeigten, vollständig durchgequollen waren und überall benetzt waren. Weiterhin wurde untersucht, ob die Gele fest, klebrig oder locker und separiert vorlagen.

Zur Durchführung des Fließtests wurden ca. 100 mg Substanz auf ein mit Wasser durchtränktes Papiertuch gegeben und verfolgt, wie das Wasser von den Produkten aufgesaugt wurde. Das Absorptionsverhalten wurde nach folgender Notenskala bewertet.
A: zieht schnell an
B: zieht sehr schnell an
C: zieht von Anfang bis Ende durch
D: Gel liegt nach der Wasseraufnahme separiert vor
E: Gelblocking

### Beispiel 1

8 g CMC Walocel 40000 (Natriumcarboxymethylcellulose, Wolff Walsrode) werden mit 2 g Favor^{R} 953 (vernetztes, teilweise neutralisiertes Natriumpolyacrylat der Fa. Stockhausen GmbH), 0,5 g TONE 230, Polyol auf der Basis von Caprolacton, Molgewicht 1 250 g/Mol (Fa. Union Carbide), 0,5 g Aerosil 200 (pyrogene Kieselsäure, Teilchendurchmesser: 12nm; Fa. Degussa) und 0,5 g Al(OH)₂OOCCH₃*1/3 H₃BO₃ unter Verwendung von 2 ml Isopropanol und 1 ml Wasser kräftig vermischt und für 60 min auf 120 °C im Ofen erhitzt.
TBT (max./ret.) = 45 g/g / 33 g/g; AUL = 9,9 g/g; FT: B C D

### Beispiel 2

60 g CMC, Walocel 40000 werden mit 1,5 g Ethylencarbonat, 1,5 ml Wasser und 1,5 ml Isopropanol homogenisiert; anschließend wird 60 min auf 120 °C im Ofen erhitzt. 8 g dieses Produktes werden mit 2 g Favor^{R} 953, 0,5 g TONE 230, 0,5 g Faser BE 600/30 (Cellulose, Durchmesser: 17 µm, Länge: 30 µm,Fa. Rettenmaier) und mit 0,5 g Al(OH)₂OOCCH₃*1/3 H₃BO₃ unter Verwendung von 2 ml Isopropanol und 1 ml Wasser kräftig vermischt und für 60 min auf 120°C im Ofen erhitzt.
TBT (max./ret.) = 46 g/g / 29 g/g; AUL = 14,4 g/g; FT: B C D

### Beispiel 3

15 g CMC, Walocel 40000 werden mit 3 g Propylencarbonat, 0,375 ml Wasser und 1,0 ml Isopropanol homogenisiert und anschließend für 60 min auf 120°C im Ofen erhitzt.
8 g des so erhaltenen Produktes werden mit 2,0 g Favor^{R} 953 ( Fa. Stockhausen GmbH), 0,5 g TONE 230, Fa. Union Carbide, 1,0 g Calciumbentonit (Fa. Südchemie) und 0,5 g Al(OH)₂OOCCH₃*1/3 H₃BO₃ versetzt sowie mit 2 ml Isopropanol und 1 ml Wasser versetzt, homogenisiert und wie zuvor thermisch nachbehandelt.
TBT (max./ret.) = 43 g/g / 27 g/g; AUL = 14,2 g/g; FT: B C D

### Beispiel 4

Es wird wie in Beispiel 3 verfahren, jedoch werden zusätzlich 0,5 g Faser BE 600/30 in das Produkt eingearbeitet.
TBT (max./ret.) = 42 g/g / 25 g/g; AUL = 15,1 g/g; FT: B C D

### Beispiel 5

2,0 g CMC, Walocel 40000, 1 g eines Polyacrylat Superabsorbers (hergestellt nach DE-P 40 15 085, Beispiel 4, im folgenden SAB "A" genannt;
Ch. F. Stockhausen GmbH), 0,25 g TONE 230, 2,25 g Faser BE 600/30 und 0,25 g Al(OH)₂OOCCH₃*1/3 H₃BO₃ werden unter Verwendung von 2 ml Isopropanol und 1 ml Wasser kräftig vermischt und für 60 Minuten auf 120°C im Ofen erhitzt.
TBT (max./ret.) = 33 g/g / 20 g/g; AUL = 14,8 g/g; FT: A D

### Beispiel 6

2,5 g CMC, Walocel 40000 werden mit 2,5 g Kelzan (Xanthan Fa. Kelco) sowie mit 1 g Favor^{R} SAB 953, 0,25 g TONE..., 0,25 g Faser BE 600/30 und 0,25 g Al(OH)₂OOCCH₃*1/3 H₃BO₃ unter Verwendung von 1 ml Isopropanol und 0.5 ml Wasser kräftig vermischt und für 60 min auf 120°C im Ofen erhitzt.
TBT (max./ret.) = 39 g/g / 22 g/g; AUL = 14,5 g/g; FT: C D

### Beispiel 7

2,5 g CMC, Walocel 30000, 2,5 g Natrosol 250 MR (Hydroxyethylcellulose, Fa. Aqualon), 1,0 g SAB "A", 0,5 g Faser BE 600/30, 0,25 g TONE 230, 0,25 g Al(OH)₂OOCCH₃*1/3 H₃BO₃ und 1,675 ml Wasser/i-Propanol (1:1) werden kräftig vermischt und anschließend 60 min auf 120°C im Ofen erhitzt.
TBT (max./ret.) = 29 g/g / 19 g/g; AUL = 13,8 g/g; FT: A C D

### Beispiel 8

2,5 g CMC 30000, 2,5 g Natrosol 250 MR (Aqualon), 1,0 g SAB "A", 0,5 g Calciumbentonit, 0,25 g TONE 230, 0,25 g Al(OH)₂OOCCH₃*1/3 H₃BO₃ und 1,675 ml Wasser/i-Propanol (1:1) werden kräftig vermischt und anschließend 60 min auf 120°C im Ofen erhitzt.
TBT (max./ret.) = 27 g/g / 23 g/g; AUL = 13,1 g/g; FT: A D

### Beispiel 9

4,0 g CMC, Walocel 30000, 0,1 g Zitronensäure, 1,0 g SAB "A", 0,25 g Faser BE 600/30, 0,25 g TONE 230 und 1,675 ml Wasser/i-Propanol (1:1) werden kräftig vermischt und anschließend 30 Minuten auf 120°C im Ofen erhitzt. In dieses Produkt werden 0,25 g Al(OH)₂OOCCH₃*1/3 H₃BO₃ eingearbeitet und für 60 Minuten auf 50°C im Ofen erhitzt.
TBT (max./ret.) = 44 g/g / 32 g/g; AUL = 11,0 g/g; FT: A C D

### Beispiel 10

Es wird wie in Beispiel 9 verfahren, jedoch wird die Faser BE 600/30 durch 0,25 g Faser (auf Basis vonPES, 3,3 dtex, 0,55 mm, Fa. Wilhelm GmbH & Co KG) ersetzt.
TBT (max./ret.) = 44 g/g / 34 g/g; AUL = 14,8 g/g;.FT: A C

### Beispiel 11

Es wird wie in Beispiel 8 verfahren, jedoch werden zusätzlich 0,5 g Faser BE 600/30 eingearbeitet.
TBT (max./ret.) = 27 g/g / 21 g/g; AUL = 13,5 g/g; FT: A C D

### Beispiele 12, 13

4,0 g CMC, Walocel 30000 werden mit 1 g SAB "A", mit 0,5 g Faser BE 600/30, mit 0,25 g TONE 230 sowie mit je = 0,1 g der in der nachfolgenden Tabelle bezeichneten Verbindung unter Hinzunahme von 1,675 ml Wasser/i-Propanol (1:1) homogenisiert und für 30 Minuten auf 120°C im Ofen erhitzt. In diese Produkte werden 0,25 g Al(OH)₂OOCCH₃*1/3 H₃BO₃ eingearbeitet und für 60 Minuten auf 60°C im Ofen erhitzt.

| Nr. | Verbindung | TBT max./ret. (g/g) | AUL (g/g) | FT |
|---|---|---|---|---|
| Beispiel 12 | GTGE ¹⁾ | 39 / 27 | 15,6 | A D |
| Beispiel 13 | EGDGE ²⁾ | 35 / 21 | 15,5 | A D |

| | | | | |
|---|---|---|---|---|
| 1) Glycerintriglycidylether | | | | |
| 2) Ethylenglykoldiglycidylether | | | | |

### Beispiel 14

8 g CMC 40000 werden mit 2 g Favor SAB 835 (vernetztes, teilweise neutralisiertes Natriumpolyacrylat der Fa. Stockhausen GmbH), 0,5 g Faser (Faser BE 600/30, Fa. Rettenmaier ), 0,5 g TONE 230, 1 ml Wasser und 2 ml i-Propanol homogenisiert und anschließend für 30 Minuten auf 120°C im Ofen erhitzt. Anschließend wird mit 0,4 g Al(OH)₂OOCCH₃*1/3 H₃BO₃ versetzt und für 60 Minuten auf 50°C im Ofen erhitzt.
TBT (max./ret.) = 45 g/g / 33 g/g; AUL = 11,4 g/g; FT: A C D

### Vergleichsbeispiel 1

Es wird wie in Beispiel 14 verfahren, jedoch wird kein Favor^{R} SAB 835 zugesetzt.
TBT (max./ret.) = 30 g/g / 27 g/g; AUL = 10,0 g/g; FT: E Dieses Produkt ist als Absorptionsmaterial ungeeignet.

### Beispiel 15

Es wird wie in Beispiel 14 verfahren, jedoch werden 0,7 g des Aluminiumsalzes zugesetzt.
TBT (max./ret.) = 47 g/g / 36 g/g; AUL = 9,4 g/g; FT: A C D

### Beispiel 16

Es wird wie in Beispiel 14 verfahren, jedoch werden 0,5 g des Aluminiumsalzes und 1 g der Faser zugesetzt.
TBT (max./ret.) = 48 g/g / 34 g/g; AUL = 9,6 g/g; FT: B C D

### Beispiel 17

8 g CMC 40000, 2 g Favor SAB 835, 0,5 g Faser BE 600/30, 0,1 g Aerosil R 972 (hydrophobierte, pyrogene Kieselsäure, Teilchendurchmesser:16 nm, Degussa AG), 1 g Wasser und 2 g Isopropanol werden homogenisiert. Dann werden 0,5 g geschmolzenes, säureterminiertes TONE 230 eingearbeitet und anschließend 30 Minuten auf 120 °C im Ofen erhitzt. Das Produkt wird mit 0,6 g des beschriebenen Aluminiumsalzes homogenisiert und anschließend für 60 Minuten auf 50°C im Ofen erhitzt.
TBT (max./ret.) = 48 g/g / 35 g/g; AUL = 10,3 g/g; FT: B C D

### Beispiel 18

Es wird wie in Beispiel 17 verfahren, jedoch wird statt des Favor^{R} SAB 835 SAB "A" verwendet.
TBT (max./ret.) = 50 g/g / 36 g/g; AUL = 11,0 g/g; FT: A C D

### Beispiel 19

8 g CMC, Walocel 40000, 2 g Favor^{R} SAB 835 , 0,5 g Faser BE 600/30, 0,25 g TONE 230, 1 g Wasser und 2 g i-Propanol werden homogenisiert und anschließend für 30 Minuten auf 120°C im Ofen erhitzt. Dannach wird mit 0,6 g Al(OH)₂OOCCH₃*1/3 H₃BO₃ versetzt und 60 Minuten auf 50°C im Ofen erhitzt.
TBT (max./ret.) = 48 g/g / 32 g/g; AUL = 9,0 g/g; FT: A C D

### Beispiel 20

Es wird wie in Beispiel 19 beschrieben verfahren, jedoch werden nur 0,1 g TONE 230 zugesetzt.
TBT (max./ret.) = 45 g/g / 30 g/g; AUL = 8,6 g/g; FT: A D

### Beispiel 21

100 g des in Beispiel 1 erhaltenen Produktes werden mit 100 ml einer 0,125 %igen wässrigen Lösung von 3,7-Bis (dimethylamino)- phenothiaziniumchlorid gemischt und anschließend für 2 Stunden im Umlufttrockenschrank bei 60°C getrocknet.
200 mg des so erhaltenen Produktes werden in einen Teebeutel gegeben. Dieser wird in ein Becherglas mit 50 ml 0,2 %iger Kochsalzlösung eingehängt. Die Färbung der Kochsalzlösung wird beurteilt, danach wird der Vorgang mit neuer NaCl-Lösung wiederholt. Nach einer Stunde wird der Teebeutel entfernt. Auch nach dem fünften Zyklus zeigt die Blaufärbung der Kochsalzlösung die Freisetzung des Wirkstoffs aus der als Speichermedium fungierenden Polymerzusammensetzung an.

### Vergleichsbeispiele 2 - 5

Die Herstellung der Produkte aus den Beispielen 1, 3, 11 und 19 wurden ohne den Zusatz von TONE 230 wiederholt. Die erhaltenen Produkte waren inhomogen, konnten durch Sieben getrennt werden und blockten. Bezüglich des TBT und des AUL-Tests konnten wegen der Inhomogenität der Produkte (Entmischung beim Absieben) keine reproduzierbaren Werte erhalten werden.

### Vergleichsbeispiel 6

20 g CMC, Walocel 30000 werden mit 8 g Isopropanol, 200 g Wasser, 0,4 g Al(OH)₂OOCCH₃*1/3 H₃BO₃ und 0,8 g Essigsäure 4 Stunden bei 50°C gehalten. Anschließend wird bei 80°C getrocknet.
TBT (max./ret.) = 16 g/g / 11 g/g; AUL = 8,9 g/g; FT: E

## Patentansprüche

1. Polymerzusammensetzung, im wesentlichen bestehend aus 70 bis 99,9 Gew.-% wenigstens einer Komponente A aus wasserlöslichen Polysacchariden und deren Derivaten, die gegebenenfalls durch Vernetzung modifiziert worden sind (Komponente A)
und
0,1 bis 30 Gew.-% wenigstens einer Komponente B aus wasserquellbaren synthetischen Polymeren und/oder Copolymeren von (Meth-)Acrylsäure, (Meth-)Acrylnitril, (Meth-)Acrylamid, Vinylacetat, Vinylpyrrolidon, Vinylpyridin, Maleinsäure(anhydrid), Itaconsäure(anhydrid), Fumarsäure, Vinylsulfonsäure und/oder 2-Acrylamido-2-methylpropansulfonsäure, den Amiden, den N-Alkylderivaten, den N,N'-Dialkylderivaten, den hydroxylgruppenhaltigen Estern und aminogruppenhaltigen Estern dieser polymerisationsfähigen Säuren, wobei 0 bis 98 Gew.-% der Säuregruppen dieser Säuren neutralisiert sind und wobei diese Polymere und /oder Copolymere durch eine mindestens bifunktionelle Verbindung vernetzt sind (Komponente B)
sowie
0,1 bis 30 Gew.-%, bezogen auf die polymeren Komponenten A und B eines organischen Matrixmaterials mit einem Schmelz- bzw. Erweichungspunkt unterhalb von 180° C zur Verhinderung der Entmischung und des Gelblocking
0,001 bis 10 Gew.-%, bezogen auf die beiden polymeren Komponenten A und B, eines ionischen und/oder kovalenten Vernetzers
und
0 bis 50 Gew.-%, bezogen auf die polymeren Komponenten A plus B, wenigstens eines Antiblockingmittels auf Basis natürlicher und/oder synthetischer Fasern und/oder Materialien mit großer Oberfläche.

2. Wirkstoffenthaltende Zusammensetzung, im wesentlichen bestehend aus einer Zusammensetzung nach Anspruch 1 sowie wenigstens einem Wirkstoff, beispielsweise einem Arzneimittel, einem Pestizid, einem Bakterizid und/oder einem Riechstoff.

3. Zusammensetzung nach Ansprüchen 1 bis 2, im wesentlichen bestehend aus
75 - 90 Gew.-% Komponente A,
10 - 25 Gew.-% Komponente B
als polymeren Komponenten und
2,5 - 7,5 Gew.-%, bezogen auf diese Komponenten, wenigstens eines Matrixmaterials,
3 - 7 Gew.-%, bezogen auf diese Komponenten, wenigstens eines ionischen oder kovalenten Vernetzters und
0,5 - 50 Gew.-%, vorzugsweie 5 - 15 Gew.-% wenigstens eines Antiblockingmittels.

4. Zusammensetzung nach Ansprüchen 1 bis 3, dadurch gekennzeichnet, daß Komponente A ein wasserlösliches und/oder wasserquellbares Polymer auf Basis von Polysacchariden und deren Derivaten, insbesondere Stärke-, Guar- und Cellulosederivaten ist.

5. Zusammensetzung nach Anspruch 4, dadurch gekennzeichnet, daß Komponente A ein anionisches Derivat von Cellulose, vorzugsweise Carboxymethylcellulose ist.

6. Zusammensetzung nach Ansprüchen 1 bis 3, dadurch gekennzeichnet, daß das Matrixmaterial bei Raumtemperatur eine hochviskose oder wachsartige weiche Konsistenz besitzt.

7. Zusammensetzung nach Anspruch 6, dadurch gekennzeichnet, daß das Matrixmaterial ausgewählt ist aus Triglycerinmonostearat, Rizinusöl und/oder Polycaprolactonen, die gegebenenfalls durch eine Umsetzung mit Maleinsäureanhydrid modifiziert sind.

8. Zusammensetzung nach Ansprüchen 1 bis 3, dadurch gekennzeichnet, daß die ionischen Vernetzer ausgewählt sind aus Metallverbindungen, vorzugsweise Magnesium-, Calcium-, Aluminium-, Zirkonium-, Eisen-, Titan- und Zinkverbindungen, in Form ihrer Salze mit organischen und anorganischen Säuren.

9. Zusammensetzung nach Ansprüchen 1 bis 3, dadurch gekennzeichnet, daß die kovalenten Vernetzer ausgewählt sind aus polyfunktionellen Carbonsäuren, Alkoholen, Aminen, Epoxyverbindungen, Carbonsäureanhydriden und/oder Aldehyden sowie deren Derivaten, sowie deren heterofunktionelle Verbindungen mit verschiedenen funktionellen Gruppen der oben genannten Verbindungsklassen.

10. Verfahren zur Herstellung der Zusammensetzung nach Ansprüchen 1 bis 9, dadurch gekennzeichnet, daß die Komponenten zunächst miteinander bis zur Homogenität vermischt und dann der Vernetzer durch die Matrix mittels einer abschließenden Wärmebehandlung fixiert wird.

11. Verfahren nach Anspruch 10, dadurch gekennzeichnet, daß man die Komponenten A und B vor dem Mischen auf eine Korngröße von 90 µm - 630 µm absiebt.

12. Verfahren nach Ansprüchen 10 oder 11, dadurch gekennzeichnet, daß man zunächst die Komponenten A und B vermischt, das Antiblockingmittel und das Matrixmaterial hierzu bis zum Entstehen einer Homogenität zumischt, indem man diese Mischung bei 25 °C bis 180 °C, vorzugsweise 100 °C bis 120 °C einer Wärmebehandlung unterzieht, und nach Zugabe des Vernetzers diesen durch die Matrix bei 25 °C bis 180 °C, vorzugsweise 50 °C bis 80 °C, fixiert.

13. Verfahren nach Ansprüchen 10 oder 11, dadurch gekennzeichnet, daß man alle Komponenten physikalisch vermischt un dann eine Wärmebehandlung bei 25 bis 180 °C, vorzugsweise 100 bis 120 °C durchführt, um den Vernetzer durch die Matrix zu fixieren.

14. Verfahren nach Ansprüchen 10 bis 13, dadurch gekennzeichnet, daß man die Komponenten vor der abschließenden Wärmebehandlung mit einem hydrophilen Lösemittel, vorzugsweise in Mengen von 1 - 10 Gew.-%, bezogen auf die Polymermischung, versetzt.

15. Verfahren nach Anspruch 14, dadurch gekennzeichnet, daß das Lösemittel Wasser oder ein Gemisch von Wasser mit wasserlöslichen organischen Lösemitteln, bevorzugt eines Alkohols mit bis zu vier C-Atomen, besonders bevorzugt ein Wasser/Isopropanol-Gemisch, ist.

16. Verfahren zur Herstellung einer Depotmaterialzusammensetzung nach Anspruch 2, dadurch gekennzeichnet, daß der Wirkstoff
- entweder aus wäßrigen oder wasserenthaltenden Lösungen von der Zusammensetzung nach Ansprüchen 1, 2 und 3 - 8 absorbiert wird und gegebenenfalls erneut getrocknet wird,
- oder als Lösung oder Dispersion bei beliebigen Vorstufen des Herstellungsverfahrens der besagten Zusammensetzung zugesetzt wird.

17. Verwendung der Zusammensetzung nach Ansprüchen 1 bis 9 oder hergestellt nach Ansprüchen 10 bis 16 als Faser, Film, Pulver oder Granulat zur Absorption von wasserenthaltenden Lösungen oder Dispersionen sowie Körperflüssigkeiten, in chemisch-technischen Produkten wie beispielsweise Verpackungsmaterialen, in Kulturgefäßen, zur Bodenverbesserung und als Kabelummantelung, in Hygienemitteln wie beispielsweise Tampons oder Windeln oder Tierhygienemitteln.

18. Verwendung der Zusammensetzung nach Ansprüchen 2 bis 8, oder hergestellt nach Anspruch 16, in Pulverform oder als Dispersion in hydrophoben Medien gegebenenfalls in Kombination mit Dispersionsstabilisatoren oder in Mischung mit anderen Stoffen.

19. (Tier) Hygienemittel enthaltend Zusammensetzungen nach Ansprüchen 1 bis 9 oder hergestellt nach Ansprüchen 10 bis 17.

20. Chemisch-technische Produkte enthaltend Zusammensetzungen nach Ansprüchen 1 bis 9 oder hergestellt nach Ansprüchen 10 bis 17.

## Claims

1. A polymer composition substantially consisting of 70 to 99.9%-wt. of at least one component A of water-soluble polysaccharides and their derivatives which have optionally been modified by cross-linkage (component A),
and
0.1 to 30%-wt. of at least one component B of water-swellable, synthetic polymers and/or copolymers of (meth-)acrylic acid, (meth-)acrylonitrile, (meth-)acrylamide, vinyl acetate, vinyl pyrrolidone, vinyl pyridine, maleic acid (anhydride), itaconic acid (anhydride), fumaric acid, vinyl sulfonic acid, and/or 2-acrylamido-2-methylpropane sulfonic acid, the amides, the N-alkyl derivatives, the N,N'-dialkyl derivatives, the hydoxyl group-containing esters and amino group-containing esters of these polymerizable acids, with 0 to 98%-wt. of the acid groups of these acids being neutralized, and these polymers and/or copolymers being cross-linked by an at least bifunctional compound (component B)
and
0.1 to 30%-wt., relative to the polymer components A and B, of an organic matrix material having a melting or softening point of below 180°C for the prevention of separation and gel blocking,
0.001 to 10%-wt., relative to the two polymer components A and B, of an ionic and/or covalent cross-linking agent,
and
0 to 50%-wt., relative to the polymer components A plus B, of at least one anti-blocking agent based on natural and/or synthetic fibers and/or large-surface materials.

2. An active substance-containing composition substantially consisting of a composition according to claim 1 and at least one active substance, for example, a drug, a pesticide, a bactericide, and/or a perfume.

3. The composition according to claims 1 to 2 substantially consisting of
75 - 90%-wt. of component A,
10 - 25%-wt. of component B
as polymer components, and
2.5 - 7.5%-wt., relative to these components, of at least one matrix material,
3 - 7%-wt., relative to these components, of at least one ionic or covalent cross-linking agent, and
0.5 - 50%-wt., preferably 5 - 15%-wt., of at least one anti-blocking agent.

4. The composition according to claims 1 to 3 characterized in that component A is a water-soluble and/or water-swellable polymer based on polysaccharides and their derivatives, in particular starch, guar, and cellulose derivatives.

5. The composition according to claim 4 characterized in that component A is an anionic derivative of cellulose, preferably carboxymethylcellulose.

6. The composition according to claims 1 to 3 characterized in that the matrix material has a highly viscous or wax-like soft consistency at room temperature.

7. The composition according to claim 6 characterized in that the matrix material is selected from triglycerol monostearate, castor oil and/or polycaprolactones, which are optionally modified by a reaction with maleic anhydride.

8. The composition according to claims 1 to 3 characterized in that the ionic cross-linking agents are selected from metallic compounds, preferably magnesium, calcium, aluminum, zirconium, iron, titanium, and zinc compounds, in the form of their salts with organic and inorganic acids.

9. The composition according to claims 1 to 3 characterized in that the covalent cross-linking agents are selected from polyfunctional carboxylic acids, alcohols, amines, epoxy compounds, carboxylic acid anhydrides, and/or aldehydes and their derivatives, as well as of their heterofunctional compounds with different functional groups of the above-mentioned compound classes.

10. A process for the production of the composition according to claims 1 to 9 characterized in that the components are mixed with one another up to homogeneity first, and that the cross-linking agent is then fixed by the matrix by means of a final heat treatment.

11. The process according to claim 10 characterized in that the components A and B are screened to a particle size of 90 µm - 630 µm, prior to mixing.

12. The process according to claims 10 or 11 characterized in that components A and B are mixed first, that the anti-blocking agent and the matrix material are mixed thereto up to homogeneity by subjecting said mixture at 25°C to 180°C, preferably 100°C to 120°C, to a heat treatment, and that, after addition of the cross-linking agent, the cross-linking agent is fixed by the matrix at 25°C to 180°C, preferably 50°C to 80°C.

13. The process according to claims 10 or 11 characterized in that all of the components are mixed physically and that then a heat treatment at 25 to 180°C, preferably 100 to 120°C, is carried out in order to fix the cross-linking agent by the matrix.

14. The process according to claims 10 to 13 characterized in that a hydrophilic solvent, preferably in amounts of 1 - 10%-wt., relative to the polymer mixture, is added to the components, prior to the final heat treatment.

15. The process according to claim 14 characterized in that the solvent is water or a mixture of water with water-soluble organic solvents, preferably an alcohol having up to four C-atoms, particularly preferred is a water/isopropanol mixture.

16. A process for the production of a depot material composition according to claim 2, characterized in that the active substance
- is either absorbed by the composition according to claims 1, 2 and 3 - 8 from aqueous or hydrous solutions and optionally dried again,
- or added to said composition as a solution or dispersion in any of the preceding stages of the production process.

17. The use of the composition according to claims 1 to 9 or manufactured according to claims 10 to 16 as fiber, film, powder, or granular material for the absorption of hydrous solutions or dispersions, and body fluids; in chemico-technical products, such as packaging materials; in culture pots; for soil conditioning, and as cable sheathing; in products of hygiene, such as tampons or diapers; or in products for animal hygiene.

18. The use of the composition according to claims 2 to 8 or manufactured according to claim 16 in the form of a powder or as dispersion in hydrophobic media, optionally in combination with dispersion stabilizers or in admixture with other substances.

19. (Animal) Hygiene means comprising compositions according to claims 1 to 9 or manufactured according to claims 10 to 17.

20. Chemico-technical products comprising compositions according to claims 1 to 9 or manufactured according to claims 10 to 17.

## Revendications

1. Composition de polymère, qui se compose, pour l'essentiel, de 70 à 99,9% en poids d'au moins un composant A formé de polysaccharides hydrosolubles et de leurs dérivés, qui ont été éventuellement modifiés par réticulation (composant A),
et
0,1 à 30% en poids d'au moins un composant B formé de copolymères et/ou de polymères synthétiques, gonflables à l'eau, de l'acide (méth)acrylique, du (méth)acrylonitrile, du (méth)acrylamide, de l'acétate de vinyle, de la vinylpyrrolidone, de la vinylpyridine, de l'acide (anhydride)maléique, de l'acide (anhydride)itaconique, de l'acide fumarique, de l'acide vinylsulfonique et/ou de l'acide 2-acrylamido-2-méthylpropanesulfonique, des amides, des dérivés N-alkyliques, des dérivés N,N'-dialkyliques, des esters contenant des radicaux hydroxyle et des esters contenant des radicaux amino, de ces acides polymérisables, où de 0 à 98% en poids des radicaux acide de ces acides sont neutralisés et où ces copolymères et/ou polymères sont réticulés par au moins un composé bifonctionnel (composant B),
ainsi que
0,1 à 30% en poids, par rapport aux composants polymériques A et B d'un matériau constituant matrice organique, possédant un point de fusion ou de ramollissement inférieur à 180°C, en vue d'empêcher la démixtion et le blocage du gel,
0,001 à 10% en poids, par rapport aux deux composants polymériques A et B, d'un agent de réticulation ionique et/ou covalent,
et
0 à 50% en poids, par rapport aux composants polymériques A plus B, d'au moins un agent antiblocage à base de fibres naturelles et/ou synthétiques et/ou de matériaux à grande surface.

2. Composition contenant des principes actifs, essentiellement constituée d'une composition selon la revendication 1, ainsi que d'au moins un principe actif, par exemple, un médicament, un pesticide, un bactéricide et/ou un parfum.

3. Composition suivant les revendications 1 ou 2, qui est essentiellement constituée de
75 à 90% en poids du composant A,
10 à 25% en poids du composant B,
à titre de composants polymériques et
2,5 à 7,5% en poids, par rapport à ces composants, d'au moins une matière constituant matrice,
3 à 7% en poids, par rapport à ces composants, d'au moins un agent de réticulation ionique ou covalent, et
0,5 à 50% en poids, de préférence, 5 à 15% en poids, d'au moins un agent antiblocage.

4. Composition suivant les revendications 1 à 3, caractérisée en ce que le composant A est un polymère hydrosoluble et/ou gonflable à l'eau, à base de polysaccharides et de leurs dérivés, plus particulièrement, des dérivés d'amidon, de gomme guar et de cellulose.

5. Composition suivant la revendication 4, caractérisée en ce que le composant A est un dérivé anionique de la cellulose, de préférence, de la carboxyméthylcellulose.

6. Composition suivant l'une quelconque des revendications 1 à 3, caractérisée en ce que la matière constituant matrice possède, à la température ambiante, une consistance extrêmement visqueuse ou cireuse molle.

7. Composition suivant la revendication 6, caractérisée en ce que l'on choisit la matière constituant matrice parmi le monostéarate de glycérine, l'huile de ricin et/ou des polycaprolactones, éventuellement modifiés par une réaction avec l'anhydride maléique.

8. Composition suivant l'une quelconque des revendications 1 à 3, caractérisée en ce que l'on choisit les agents de réticulation ioniques parmi des composés de métaux, de préférence, des composés du magnésium, du calcium, de l'aluminium, du zirconium, du fer, du titane et du zinc, sous forme de leurs sels avec des acides organiques et inorganiques.

9. Composition suivant les revendications 1 à 3, caractérisée en ce qu'on choisit les agents de réticulation covalents parmi des aldéhydes, des anhydrides d'acides carboxyliques, des composés époxy, des amines, des alcools, des acides polyfonctionnels, comme aussi leurs dérivés, ainsi que leurs dérivés hétéro-fonctionnels avec divers groupes fonctionnels appartenant aux classes de composés susmentionnées.

10. Procédé de préparation de la composition suivant l'une quelconque des revendications 1 à 9, caractérisé en ce que l'on mélange d'abord les composants les uns aux autres jusqu'à homogénéité et on fixe ensuite l'agent de réticulation à travers la matrice à l'aide d'un traitement thermique subséquent.

11. Procédé suivant la revendication 10, caractérisé en ce que l'on sépare par criblage les composants A et B avant le mélange jusqu'à une granulométrie de 90 µm-630 µm.

12. Procédé suivant la revendication 10 ou 11, caractérisé en ce que l'on mélange d'abord les composants A et B, on y incorpore ensuite sous mélange l'agent antiblocage et la matière constituant matrice jusqu'à obtention d'une homogénéité, pour autant que l'on soumette ce mélange à un traitement thermique à 25°C-180°C, de préférence, 100°C à 120°C et que, après l'addition de l'agent de réticulation, on fixe celui-ci à travers la matrice à 25°C-180°C, de préférence, 50°C à 80°C.

13. Procédé suivant les revendications 10 ou 11, caractérisé en ce que l'on mélange physiquement tous les composants et on entreprend ensuite un traitement thermique à 25-180°C, de préférence, 100-120°C, afin de fixer l'agent de réticulation à travers la matrice.

14. Procédé suivant les revendications 10 à 13, caractérisé en ce que l'on ajoute un solvant hydrophile, de préférence en proportions de 1 à 10% en poids, par rapport au mélange de polymères, aux composants avant le traitement thermique subséquent.

15. Procédé suivant la revendication 14, caractérisé en ce que le solvant est l'eau ou un mélange d'eau et de solvants organiques hydrosolubles, de préférence, un alcool qui compte jusqu'à 4 atomes de carbone, de manière particulièrement avantageuse, un mélange d'eau et d'isopropanol.

16. Procédé de préparation d'une composition de matière dépôt selon la revendication 2, caractérisé en ce que
- on absorbe le principe actif à partir de solutions aqueuses ou contenant de l'eau de la composition suivant les revendications 1, 2 et 3-8 et on opère un éventuel nouveau séchage,
- ou bien on ajoute le principe actif sous forme de solution ou de dispersion au cours de n'importe quelles étapes préalables du procédé de préparation de la composition précitée.

17. Utilisation de la composition suivant l'une quelconque des revendications 1 à 9, ou préparée selon l'une quelconque des revendications 1 à 16, sous forme de fibre, de film, de poudre, ou de granulé, en vue de l'absorption de dispersions ou de solutions contenant de l'eau, ainsi que de liquides corporels, dans des produits chimicotechniques, tels que, par exemple, des matériaux d'emballage, dans des récipients de culture, pour l'amélioration ou l'amendement du sol et à titre d'enrobage de câble, dans des agents d'hygiène, comme, par exemple, des tampons, ou des couches, ou des agents pour l'hygiène animale.

18. Utilisation de la composition suivant l'une quelconque des revendications 2 à 8, ou préparée selon la revendication 16, sous forme de poudre ou sous forme de dispersion dans des milieux hydrophobes, éventuellement en combinaison avec des stabilisateurs de dispersion, ou en mélange à d'autres substances

19. Compositions contenant des agents d'hygiène (animale) selon les revendications 1 à 9, ou préparées selon les revendications 10 à 17.

20. Produits chimicotechniques, qui contiennent des compositions selon l'une quelconque des revendications 1 à 9 ou préparés selon les revendications 10 à 17.
